# EUROPEAN PATENT APPLICATION

(11) **EP 4 691 552 A1**
(43) Date of publication of application: **11.02.2026**
(21) Application number: 24193118.7
(22) Date of filing: 06.08.2024
(51) Int. Cl.: A61N 5/10

(54) **COMPUTER-IMPLEMENTED METHOD, COMPUTER PROGRAM PRODUCT, APPARATUS AND SYSTEM FOR RADIATION TREATMENT PLANNING**

(71) Applicant: Siemens Healthineers International AG, 6312 Steinhausen (CH)
(72) Inventor: PATRICIO, Pedro, 8005-226 Faro (PT)
(74) Representative: HKW Intellectual Property PartG mbB

(57) **Abstract**

The invention relates to a computer-implemented method, a computer-implemented apparatus, a system and a computer program product for radiation treatment planning comprising determining a radiation treatment plan comprising a number N, with N ≥ 1, of radiation sequences to be applied to a patient, each radiation sequence being associated with a radiation dose rate, determining a maximum radiation dose rate of the radiation treatment plan, the maximum radiation dose rate being associated with one of the N radiation sequences and configuring a radiation apparatus to apply an adapted maximum radiation dose rate in accordance with the determined maximum radiation dose rate, wherein the adapted maximum radiation dose rate is less than an available maximum radiation dose rate at the radiation apparatus.

## Description

The present invention relates to a computer-implemented method, a computer program product, an apparatus and a system for radiation treatment planning. The increasing number of cancer diagnoses every year increased the demand for modern treatment facilities that are capable of providing an increasing number of patients access to adequate treatment schemes.

A commonly used treatment method for cancer therapy is radiation therapy wherein tumor cells are at least locally irradiated by a suitable radiation beam such as, e.g., X-rays, gamma rays, protons, heavy ions, etc. A critical aspect of radiation therapy is the radiation dose required to effectively harm malign tumor cells while ensuring that the radiation dose deployed in healthy tissue is minimized (e.g., to avoid the risk of causing carcinogenic cell defects as a result of the radiation process) while keeping the overall duration (e.g., the total time required to ensure that a pre-defined radiation dose is arriving at a malign tissue of a patient) of the radiation process as low as possible. This may in particular ensure that the time required to ensure that a certain radiation dose arrives at the patient is minimized. As a result, the risk that the patient may move unexpectedly (which may cause an undesired irradiation of healthy tissue) during a radiation procedure may thus be reduced.

An actual radiation therapy is carried out based on a preceding radiation treatment planning during which a dose required to arrive at a patient (i.e., an upper radiation dose rate that effectively arrives at the patient) is determined for a plurality of settings of a radiation apparatus (e.g., various directions under which a radiation beam may impinge onto a patient, a meterset weight setting, collimation settings, multileaf positions, dose rate, gantry speed, rotation angle of a gantry, etc.).

A radiation apparatus may comprise a radiation particle source (e.g., for electrons, protons, heavy ions, X-rays or gamma rays, etc.), an acceleration stage and/or may comprise a gantry for directing the accelerated radiation particles onto a patient. A radiation apparatus may in many cases be operated at an available maximum radiation dose (e.g., as a default setting) even though the available maximum radiation dose is not necessarily required as determined by the preceding radiation treatment planning.

Even though radiation therapy is frequently applied in hospitals and/or dedicated practices, radiation therapy remains a cost-intensive method for cancer therapy. This cost-intensiveness is, e.g., driven by the required medical staff (e.g., for planning the radiation therapy and/or executing the actual radiation therapy), the costs for the required hardware (e.g., a radiation apparatus and potential replacement parts), maintenance costs, energy costs for operating the hardware, etc.

Since a radiation apparatus may oftentimes be operated at an available maximum radiation dose rate, even though a smaller dose rate is required at the patient, energy required for providing the available maximum radiation dose rate at the radiation apparatus may unduly be wasted. Over the course of many years of operating the radiation apparatus, these wasted energy may lead to a significant amount of money that may be considered wasted and which could, e.g., be used for improving the general infrastructure of a hospital and/or other treatment improvements instead. Furthermore, this undue energy demand may affect the carbon footprint of radiation therapies in a negative and disadvantageous manner. What is more, operating the radiation apparatus at the available maximum radiation dose per default may also lead to unnecessarily high strain on the electrical components of the radiation apparatus (e.g., a thyratron) that may be associated with operating the radiation apparatus at the available maximum radiation dose. As a result, the strained electrical components may arrive at their expected end of life sooner, may thus be replaced sooner and/or more frequently and may thus lead to higher operational costs of the radiation apparatus and consequently also to higher costs of the radiation therapy as such.

Therefore, a radiation treatment planning and a subsequently executed radiation therapy that takes these aspects into account may not always be possible in a satisfying manner.

It is one object to provide a method and a corresponding apparatus for radiation treatment planning that tailors a configuration of a radiation treatment apparatus more closely to the actual requirements set out in a respective radiation treatment plan.

According to a first aspect, a computer-implemented method for radiation treatment planning is suggested. The method comprises determining a radiation treatment plan comprising a number N, with N ≥ 1, of radiation sequences to be applied to a patient, each radiation sequence being associated with a radiation dose rate and determining a maximum radiation dose rate of the radiation treatment plan, the maximum radiation dose rate being associated with one of the N radiation sequences. Moreover, the method comprises configuring a radiation apparatus to apply an adapted maximum radiation dose rate in accordance with the determined maximum radiation dose rate, wherein the adapted maximum radiation dose rate is less than an available maximum radiation dose rate at the radiation apparatus.

A maximum radiation dose rate may be understood as the maximum radiation dose rate that has been determined as part of the determining of the radiation treatment plan. That is, the determined radiation treatment plan may comprise a plurality of different radiation dose rates for, e.g., different settings of the radiation apparatus wherein the maximum radiation dose rate may represent the maximum radiation dose rate comprised by the radiation treatment plan.

The adapted radiation dose rate may be understood as a radiation dose rate that lies closest to the determined maximum radiation dose rate. In some cases, the radiation apparatus may provide a set of radiation dose rates at which the radiation apparatus may be operated (e.g., 500 meterweight units (MU)/min, 700 MU/min, 800 MU/min, 900 MU/min, 1000 MU/min, 1200 MU/min, 1500 MU/min, etc.). That is, the determined maximum radiation dose rate may, e.g., be 977 MU/min whereas the radiation apparatus may be set to one of a set of possible radiation dose rates (e.g., 500 MU/min, 700 MU/min, 800 MU/min, 900 MU/min, 1000 MU/min, 1200 MU/min, 1500 MU/min, etc.). In such an exemplary case, the adapted maximum radiation dose rate may be set to 1000 MU/min as it represents the maximum radiation dose rate that lies closest to the determined maximum radiation dose rate and which is still larger than the determined maximum radiation dose rate.

The available maximum radiation dose rate may be understood as the maximum radiation dose rate the radiation apparatus is technically capable of supplying. This may at least partially arise due to internal restrictions of the radiation apparatus, e.g., due to maximum currents/voltages internal components of the radiation apparatus may be capable of handling. The available maximum radiation dose rate may correspond to a maximum design dose rate the radiation apparatus is capable of handling. This restriction may, e.g., be based at least in part on a maximum current the radiation apparatus may (internally) be capable of handling.

It will be appreciated that a time integral over an applied radiation dose rate and the temporal length of, e.g., a radiation sequence may lead to the (total) dose arriving at the patient in said time interval. On the other hand, a derivative of the total dose, when taking the temporal duration of the radiation sequence into account, may yield a respective average radiation dose rate delivered in the respective time interval.

Additionally or alternatively to the configuring of the radiation apparatus to apply the adapted maximum radiation dose rate, the computer-implemented method may comprise controlling the radiation apparatus to apply the adapted maximum radiation dose rate. In this case, the computer-implemented method may further comprise sending respective control commands to the radiation apparatus that control the radiation apparatus to generate the adapted maximum radiation dose rate.

By configuring the radiation apparatus to apply the adapted maximum radiation dose rate which may be lower than the available maximum radiation dose rate, the radiation dose rate provided by the radiation apparatus may be decreased. This decrease may be accompanied by a decrease of the energy consumption of the radiation apparatus and may thus contribute to more energy efficient radiation therapies originating from an improved radiation treatment planning. Therefore, the energy costs of a radiation therapy may be decreased. Moreover, the stress on electrical components that are associated with providing a certain radiation dose rate may efficiently be decreased and the expected lifetime of these components (e.g., of a thyratron) may be extended. This may at least in part arise from the aspect that less heat may be produced at the thyratron if the radiation apparatus is operated at the adapted maximum radiation dose rate as compared to an operation of the radiation apparatus at the available maximum radiation dose rate. Consequently, the operation costs of the radiation apparatus may efficiently be decreased. Moreover, maintenance periods associated with a replacement of worn electrical parts may be minimized and generally suppressed. As a synergetic effect, the environmental footprint associated with operating the radiation apparatus may be reduced.

According to an embodiment, each of the N radiation sequences may be associated with a gantry angle of a set of gantry angles of the radiation apparatus.

A radiation sequence may be understood as a time period during which a certain radiation dose (e.g., defined by the radiation treatment plan) arrives at the patient based on a certain radiation dose rate generated by the radiation apparatus. The time period may, e.g., be defined by a time interval during which one or more settings of the radiation apparatus may be kept constant (such as, e.g., a meterset weight, a collimation setting, dose rate setting, a gantry speed, a meterset weight/degree setting, a multileaf position, etc.). Additionally or alternatively, a radiation sequence may be defined by an angular interval of the gantry (e.g., the period of time required for the gantry to, e.g., rotate by an angular sequence of 1-2°).

A radiation treatment plan may, e.g., be understood as a list of radiation doses and/or radiation dose rates that are to be applied to the patient by means of the radiation apparatus. A radiation dose may be associated with at least one configuration parameter of the radiation apparatus (e.g., a certain gantry angle of the radiation apparatus, a bed angle of the radiation apparatus, a collimator configuration, etc.).

As a result, a fine granular configuration of the radiation therapy for the patient may be facilitated.

According to a further embodiment, the determining of the N radiation sequences may comprise determining a first number M, with M ≥ 1, of gun pulses defining a first temporal sequence at which charged particles enter an acceleration stage of the radiation apparatus and determining a first number K, with K ≥ 1, of modulator pulses defining a first temporal sequence at which the charged particles are accelerated in the acceleration stage of the radiation apparatus.

In some examples, the charged particles may be positively charged. Alternatively, the charged particles may be negatively charged. In some examples, the charged particles may be electrons, protons, heavy ions and/or any other suitable type of charged particles.

In some examples, the charged particles may interact with a target medium that converts the impinging charged particles into X-rays and/or gamma rays wherein the X-rays and/or gamma rays are subsequently used for providing the patient with a certain radiation dose rate in accordance with the determined radiation treatment plan.

The gun pulses may be understood as those pulses which may lead to an injection of charged particles into the acceleration stage. In some examples, the gun pules may be associated with a number of pulses per predefined time interval at which charged particles may be injected into the acceleration stage of the radiation apparatus.

The modulator pulses may be understood as those pulses which may lead to an acceleration of the injected charged particles along the acceleration stage. That is, a modulator pulse may be associated with a voltage and/or current pulse that may travel along a set of electrodes (the modulator pulse charging the respective electrodes) arranged along a longitudinal direction of the acceleration stage, wherein each of the electrodes may be configured to accelerate charged particles along the longitudinal direction of the acceleration stage.

Electrodes in the present context may refer to electric field applying means (e.g., made from metal and/or conducting plastics and/or any other suitable material) which may exhibit an electric field that may accelerate charged particles along the longitudinal direction of the acceleration stage.

The gun pulses and the modulator pulses may be coincident in time. In some cases, a modulator pulse may be provided such that a duration of the modulator pulse is at least equal to the duration of a gun pulse (or such that a duration of the modulator pulse is longer than a duration of a gun pulse). In some examples, at least some of the gun pulses and the modulator pulses may not be coincident in time. It is emphasized that in scenarios in which gun pulses and modulator pulses do not overlap, no acceleration of charged particles occurs. Consequently, no radiation dose is generated and no radiation dose arrives at the patient under these circumstances.

The gun pulses and/or the modulator pulses may be equidistant in time (i.e., may be temporally separated from each other by a same temporal interval such as, e.g., 1 ms, 2 ms, 3 ms, 4 ms, 5 ms, 6 ms, 7 ms, 8 ms, 9 ms or 10 ms. In some examples, the gun pulses and/or the modulator pulses may be separated from each other by 2.7 ms or 5.5 ms.

The determining of a first number M of gun pulses defining a first temporal sequence at which charged particles may enter an acceleration stage and of a first number K of modulator pulses defining a first temporal sequence at which charged particles are accelerated may allow a precise definition of each of the radiation sequences and may allow an efficient definition of those settings of the radiation apparatus that may be causal, when applied accordingly, for providing a certain radiation dose rate to the patient in accordance with the determined radiation treatment plan.

According to a further embodiment, the configuring may comprise determining a second number M of gun pulses defining a second temporal sequence at which charged particles enter the acceleration stage and/or a second number K of modulator pulses defining a second temporal sequence at which the charged particles are accelerated, wherein the second temporal sequence of gun pulses and/or the second temporal sequence of modulator pulses comprises less pulses in a predefined time interval than the respective first temporal sequence of gun pulses and/or the first temporal sequence of modulator pulses.

By determining a second temporal sequence that comprises less pulses in a predefined time interval as compared to a respective first temporal sequence, the energy required to execute the generation of the second temporal sequence in the radiation apparatus may be decreased as compared to the respective first temporal sequence. This may essentially be achieved by changing a frequency at which the gun pulses and/or of the modulator pulses are produced in the respective second temporal sequence as compared to the respective first temporal sequence. As a consequence, a total energy consumption associated with producing the second temporal sequence may be lowered as compared to the total energy consumption associated with producing the first temporal sequence.

Moreover, the transmitted radiation dose rate associated with the second temporal sequence may be decreased as compared to a respective first temporal sequence. Consequently, also the transmitted radiation dose may be decreased if the temporal duration of the respective radiation sequence is maintained.

According to a further embodiment, the second number M of gun pulses may define a second temporal sequence at which charged particles enter the acceleration stage that may be lower as compared to the first number M of gun pulses defining a first temporal sequence at which charged particles enter the acceleration stage and/or wherein the second number of modulator pulses may define a second temporal sequence at which charged particles are accelerated may be lower as compared to the first number K of modulator pulses defining a first temporal sequence at which charged particles are accelerated.

In some cases, the second number M of gun pulses may define a second temporal sequence at which charged particles enter the acceleration stage that may be lower by at least a factor of 5% - 95%, preferably by 10% - 80%, more preferably by 15% - 60% and most preferably by 20% - 50% as compared to the first number M of gun pulses defining a first temporal sequence at which charged particles enter the acceleration stage.

Additionally or alternatively the second number K of modulator pulses may define a second temporal sequence at which charged particles are accelerated may be lower by at least a factor of 5% - 95%, preferably by 10% - 80%, more preferably by 15% - 60% and most preferably by 20% - 50% as compared to the first number K of modulator pulses defining a first temporal sequence at which charged particles are accelerated.

As a result, the energy demand required for generating the respective second temporal sequence may be lowered if only the number K of modulator pulses is lowered. Consequently, an energy efficient operation of the radiation apparatus may be supported.

According to a further embodiment, an acceleration of charged particles that enter the acceleration stage may occur when a gun pulse of the number M of gun pulses at least partially overlaps in the time domain with a modulator pulse of the number K of modulator pulses.

In some examples, the gun pulses of the number M of gun pulses and the modulator pulses of the number K of modulator pulses may be synchronized.

An at least partial overlap of the gun pulses of the number M of gun pulses and the modulator pulses of the number K of modulator pulses may support an efficient acceleration of charged particles that have entered the acceleration stage.

According to a further embodiment, determining the maximum radiation dose rate of the radiation treatment plan may comprise determining a particular temporal sequence of gun pulses and/or a temporal sequence of modulator pulses that may cause the determined maximum radiation dose rate.

The determined temporal sequence of gun pulses and/or the determined temporal sequence of modulator pulses may be causal for the determined maximum radiation dose rate.

Based thereon, if the determined temporal sequence is a first number M of gun pulses defining a first temporal sequence at which charged particles enter the acceleration stage and/or a first number K of modulator pulses defining a first temporal sequence at which charged particles are accelerated, a starting condition regarding the pulse setting which are causal for a current energy consumption and/or radiation dose may be defined based on which a decreased second number M of gun pulses defining a second temporal sequence at which charged particles may enter the acceleration stage and/or a second number K of modulator pulses defining a second temporal sequence at which charged particles are accelerated may be determined based thereon.

According to a further embodiment, each radiation dose applied to a patient according to the radiation treatment plan and as derived from each of the K radiation sequences may be equal to a radiation dose associated with the adapted maximum dose the radiation apparatus is configured with.

That is, even though the radiation apparatus may be operated at a radiation dose rate that is lower than an available maximum radiation dose rate, the radiation dose applied to a patient (i.e., the radiation dose that arrives at the patient) is unchanged and is still in accordance with the required radiation dose according to radiation treatment plan. This comes about the fact that even though the radiation apparatus may oftentimes be operated at its available maximum radiation dose rate (per default), this available maximum radiation dose rate may seldomly arrive at the patient. This limitation is, e.g., based on the limited rotation speed of the gantry which may, e.g., at most be 6°/second.

According to a further embodiment, the method may further comprise configuring the radiation apparatus to apply the radiation sequence in accordance with the configured adapted maximum radiation dose rate and the determined radiation treatment plan.

By effectively applying the radiation sequence in accordance with the configured adapted maximum radiation dose rate and the determined radiation treatment plan, an energy and operational efficient radiation therapy may be provided to the patient.

According to a further embodiment, the configured adapted maximum radiation dose rate of the radiation apparatus may at least be lower by 30%, preferably by at least 40%, more preferably by at least 60% and most preferably by at least 70% than the available maximum radiation dose rate of the radiation apparatus.

As a result, a significant reduction of the energy consumption associated with the radiation therapy and a significant extension of the lifetime of stressed electrical components (associated with providing a respective radiation dose) may be achieved.

According to a further embodiment, the radiation treatment planning may be performed for a radiation therapy of a breast, a prostate, a head and/or a neck of the patient.

Consequently, a versatile radiation therapy may be provided to the patient while taking advantage of the achievable energy efficiency according to aspects of the present invention.

According to a second aspect, a computer program product is suggested. The computer program product may comprise instructions which, when the program is executed by a computer, cause the computer to carry out the method according to any of the embodiments described herein.

A computer program product, such as a computer program means, may be embodied as a memory card, USB stick, CD-ROM, DVD or as a file which may be downloaded from a server in 10 a network. For example, such a file may be provided by transferring the file comprising the computer program product from a wireless communication network.

According to a third aspect, a computer-implemented apparatus for radiation treatment planning is suggested. The apparatus may comprise a determining unit for determining a radiation treatment plan comprising a number N, with N ≥ 1, of radiation sequences to be applied to a patient, each radiation sequence being associated with a radiation dose, a further determining unit for determining a maximum radiation dose rate of the radiation treatment plan, the maximum radiation dose rate being associated with one of the N radiation sequences and a configuring unit for configuring a radiation apparatus to apply an adapted maximum radiation dose rate in accordance with the determined maximum radiation dose rate, wherein the adapted maximum radiation dose rate is less than an available maximum radiation dose rate at the radiation apparatus.

The determining unit, the further determining unit and the configuring unit may be provided by any means that are capable of fulfilling the demands set for the respective units.

The radiation apparatus may comprise a particle source (e.g., an electron source, a proton source, a heavy ion source, etc.) such as e.g., a particle gun, an acceleration stage which may accelerate particles, e.g., close to the speed of light and a gantry which may be configured to direct the accelerated particles (and/or X-rays or gamma rays into which the accelerated particles may have been converted to) onto the patient and in accordance with the determined radiation treatment plan.

According to an embodiment, the computer-implemented apparatus may further comprise an execution unit for executing the computer-implemented method according to one of the embodiments described herein and/or a further execution unit for executing the computer program product as described above. The execution unit and the further execution unit may be provided by any means that are capable of fulfilling the demands set for the respective units.

According to a fourth aspect, a system for radiation treatment planning may comprise the computer-implemented apparatus as described herein and the computer program product as described herein.

In some examples, the computer-implemented apparatus and the computer program product may be provided as a single device. Alternatively, the computer-implemented apparatus and the computer program product may be arranged as two separate devices. In the latter case, the computer program product may, e.g., be arranged at a remote unit such as, e.g., a remote server, a remote database, etc.

In one or more examples, the functions described may generally be implemented in hardware, software, firmware, or any combination thereof. If implemented in software, the functions may be stored on or encoded as one or more instructions or code on a computer- readable medium. Computer-readable media includes computer storage media. Storage media may be any available media that can be accessed by a computer. By way of example, and not limitation, such computer-readable media can comprise random-access memory (RAM), read-only memory (ROM), electronically erasable programmable ROM (EEPROM), compact disk (CD) ROM (CD-ROM), or other optical disk storage, magnetic disk storage or other magnetic storage devices, or any other medium that can be used to carry or store desired program code in the form of instructions or data structures and that can be accessed by a computer. Disk and disc, as used herein, includes CD, laser disc, optical disc, digital versatile disc (DVD), and floppy disk where disks usually reproduce data magnetically, while discs reproduce data optically with lasers. Combinations of the above should also be included within the scope of computer readable media.

The embodiments and features described with reference to the apparatus of the present invention apply *mutatis mutandis* to the method of the present invention and vice versa.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

The respective unit, e.g. the determining unit, may be implemented in hardware and/or in software. If said unit is implemented in hardware, it may be embodied as a device, e.g. as a computer or as a processor or as a part of a system, e.g. a computer system. If said unit is implemented in software it may be embodied as a computer program product, as a function, as a routine, as a program code or as an executable object.

Any embodiment of the first aspect may be combined with any embodiment of the first aspect to obtain another embodiment of the first aspect.

Further embodiments, features and advantages of the present invention will become apparent from the subsequent description and dependent claims, taken in conjunction with the accompanying drawings, in which:
Fig. 1 shows an exemplary system implementing a computer-implemented apparatus according to aspects of the present invention;
Fig. 2 shows an exemplary radiation apparatus according to aspects of the present invention;
Fig. 3 depicts an exemplary modulator pulse (top) and an exemplary gun pulse (bottom) in the time domain;
Fig. 4 depicts an exemplary dose distribution diagram indicating a radiation dose arriving at a patient for different gantry angles of the gantry of a radiation apparatus;
Figs. 5A and 5B depict an exemplary relationship between a dose rate arriving at a patient and a rotation speed of a gantry;
Fig. 6 depicts an exemplary comparison between the interplay of modulator pulses and gun pulses in the time domain;
Fig. 7 shows an exemplary computer-implemented method for radiation treatment planning;
Fig. 8 shows an exemplary computer-implemented apparatus for radiation treatment planning; and
Fig. 9 shows an exemplary system for radiation treatment planning.

In the Figures, reference numerals designate like or functionally equivalent elements, unless otherwise indicated.

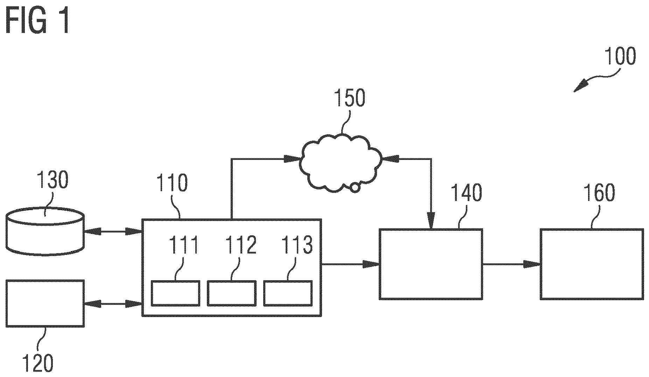

Fig. 1 shows an exemplary system 100 implementing aspects of the present invention.

System 100 comprises a computer-implemented apparatus 110 as described elsewhere herein. The computer-implemented apparatus 110 comprises a determining unit 111 as described elsehwhere herein. The computer-implemented apparatus 110 may comprise a further determining unit 112 for automatically determining a maximum radiation dose rate as described elsewhere herein. Moreover, the computer-implemented apparatus 110 may further comprise a configuring unit 113 for configuring a radiation apparatus 140 as described elsewhere herein.

Computer-implemented apparatus 110 may be in (bidirectional) communication (e.g., wired and/or wireless) with a user interface 120. User Interface 120 may be provided as a (touch-senstive) screen, a speech-to-text conversion unit, a handheld device (e.g., a tablet, a notebook, a mobile phone, etc.) which may be adapted to receive a user input received from a user of the system 100. Additionally or alternatively, the user interface 120 may be configured to receive one or more commands from the computer-implemented apparatus 110 and may be adapted to provide the received commands as information to the user of the system 100. In some exeamples, the user interface 120 may thus display a prompt to the user of the system 100 requesting information associated with a treatment planning process (as described elsewhere herein).

Computer-implemented apparatus 110 may be in (bidrectional) communication (e.g., wired and/or wireless) with a remote unit 130. Remote unit 130 may act as a remote storage facility, e.g., a database confgured to store information that may be associated with an radiation treatment planning according to aspects of the present invention. That information may, e.g., comprise information associated with a previously performed radiation treatment planning, CT and/or X-ray scans associated with a patient to be irradiated. In some examples, remote unit 130 may be configured to store a (hospital) patient file and/or any other patient-related data that may be useful for radiation treatment planning. In some examples, remote unit 130 may store one or more previously generated and/or used radiation treatment plans.

Computer-implemented apparatus 110 may further be in communication with the radiation apparatus 140. Radiation apparatus 140 may be an apparatus capable of providing a radiation beam such as, e.g., X-rays, gamma rays, protons, (charged) heavy ions and/or any other suitable radiation source that may be capable of providing a radiation beam for the radiation of malign tissue. Radiation apparatus 140 may comprise a gantry that may be rotated about a patient to be irradiated who may preferably be placed at a center region of the gantry. In some examples, the radiation apparatus 140 may comprise a collimator (e.g., a multi-leaf collimator) adapted to shape the radiation beam.

Radiation apparatus 140 may be configured to perform a radiation process in accordance with a configuration received from the computer-implemented apparatus 110. In some examples, the configuration may comprise one or more control points for controlling a movement/rotation of the gantry as described elswhere herein.

Radiation apparatus 140 may generate a radiation (beam) in accordance with the received configuration and may be configured to direct the generated radiation beam onto a patient 160 to be irradiated.

In some examples, computer-implemented device 110 may additionally or alternatively be in communication with a cloud service 150 that may be hosted at a remote server and/or a hospital server and/or at any other suitable entity. Cloud service 150 may be in (bidirectional) communication with the radiation apparatus 140. Cloud service 150 may be configured to receive a configuration for the radiation apparatus 140 as provided by the computer-implemented device 110 and may at least temporarily store the configuration, e.g., in a database. Cloud service 150 may be acessible, e.g., by a physician by means of an internet connection and/or a local area network.

Cloud service 150 may be configured to relay the received configuration to the radiation apparatus 140.

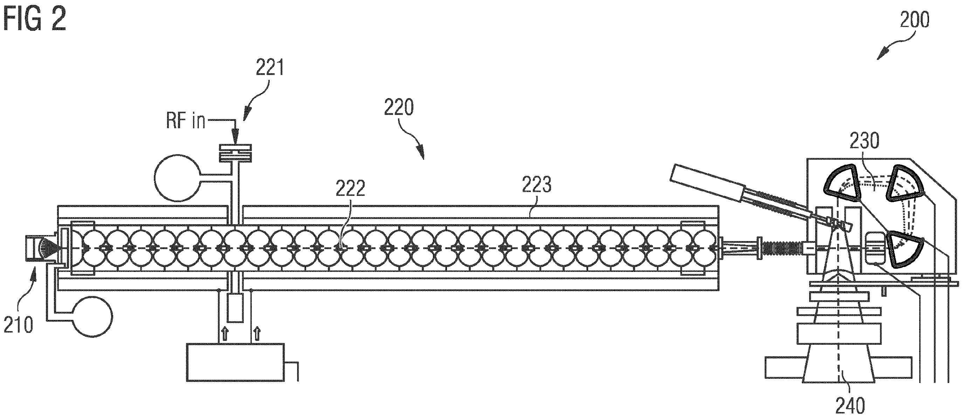

Fig. 2 shows an exemplary radiation apparatus 200. Radiation apparatus 200 may be configured identically to radiation apparatus 140 as described above.

Radiation apparatus 200 may comprise a gun 210. Gun 210 may be configured to release one or more charged particles such as electrons, protons, heavy ions and/or any other suitable sort of charged particles.

The released one or more charged particles may be injected into acceleration stage 220 that may be connected subsequently to the gun 210.

Acceleration stage 220 may comprise an RF input unit 221, one or more acceleration electrodes 222 and a solenoid 223 circumferent to the one or more electrodes 222 and the acceleration stage 220 along a longitudinal direction of the acceleration stage 220.

RF input unit 221 may, e.g., comprise a thyratron that may be configured to generate a certain number K of modulator pulses. In some examples, the RF input unit 221 may thus provide 100 to 500 pulses/s (also: 100 to 500 Hz), preferably 200 to 400 pulses/s, more preferably 300 to 380 pulses/s and most preferably 360 pulses/s if the radiation apparatus 200 is operated at the available maximum radiation dose rate. It may also be possible that less than 100 pulses/s (such as, e.g., 20 pulses/s, 50 pulses/s, 80 pulses/s, etc.) are provided by the RF unit 221.

In an example, if the RF input unit 221 is operated at 360 pulses/s and if the number K of modulator pulses is lowered from a first number of modulator pulses to 50% thereof, the second number K of modulator pulses may thus be 180 pulses/s.

The acceleration electrodes 222 may be arranged subsequently to each other along a longitudinal extension of the acceleration stage 200. The acceleration electrodes 222 may be arranged equidistantly from each other. If a charged particle (e.g., an electron but not limited thereto) is injected into the acceleration stage 220, the charged particle may be accelerated between each of two subsequently arranged acceleration electrodes 222 according to the principle of a linear accelerator and driven by the modulator pulses of the RF input unit 221.

Solenoid 223 may be arranged circumferent to the acceleration stage 220 and may, when operational, provide a guiding field that guides the accelerated particles along the longitudinal direction of the acceleration stage 220.

Acceleration stage 220 may be followed by a beam bending unit 230. Beam bending unit 230 may be configured such that accelerated electrons entering the beam bending unit 230 from the acceleration stage 220 may be stirred such that they may enter a beam shaping unit 240. Beam bending unit 230 may comprise one or more bending magnets (e.g., quadrupole magnets) that may be configured to apply a Lorentz force onto the incident electron beam and may thus change the direction of motion of the electron beam. As a result, the accelerated electrons may enter the beam shaping unit 240 in a controlled manner.

Beam shaping unit 240 may comprise one or more beam shaping optics (not shown in Fig. 2) that may be adapted to shape the accelerated electron beam in a way that it fulfills the requirements of the beam for a radiation therapy.

In some examples, the beam shaping unit 240 may be followed by a cathode (e.g., an anti-cathode, not shown in Fig. 2) onto which the accelerated electrons may impinge. Based thereon, bremsstrahlung (i.e., X-rays) which may be used to irradiate respective malign tissue of the patient may be generated.

In some cases, the beam shaping unit 240 and the cathode may also be arranged in reversed order. In such a case, the beam shaping unit 240 may comprise a flattening filter.

The interior of the radiation apparatus 200 may be evacuated.

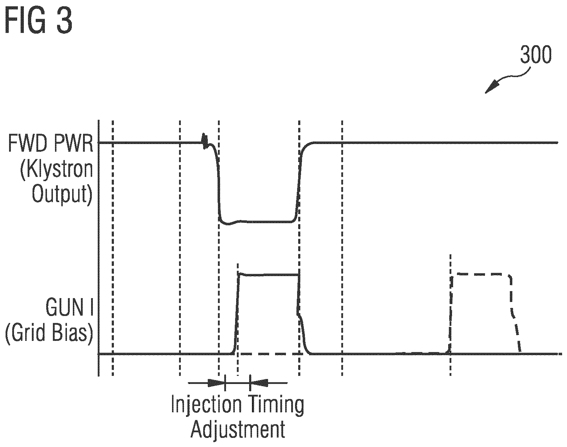

Fig. 3 depicts exemplary pulses 300. More specifically, Fig. 3 depicts a modulator pulse (top) and an exemplary gun pulse (bottom) in the time domain.

In some examples, the modulator pulse (e.g., a modulator pulse of the number K of modulator pulse) and/or the gun pulse (e.g., a gun pulse of the number M of modulator pulses) may be provided as step pulse. In other examples, the modulator pulse and/or the gun pulse may be provided as a sawtooth pulse, a sinusoid, a ramp pulse and/or with any other suitable pulse shape.

A temporal extension of the modulator pulse and/or the gun pulse may, e.g., be between 6 to 26 µs, most preferably of 7.8 µs.

The temporal separation between two subsequently arranged modulator pulses and/or gun pulses may, e.g., be in between 2.5 ms and 10 ms, In some cases, the temporal separation may also lie in between 6 µs and 10 ms.

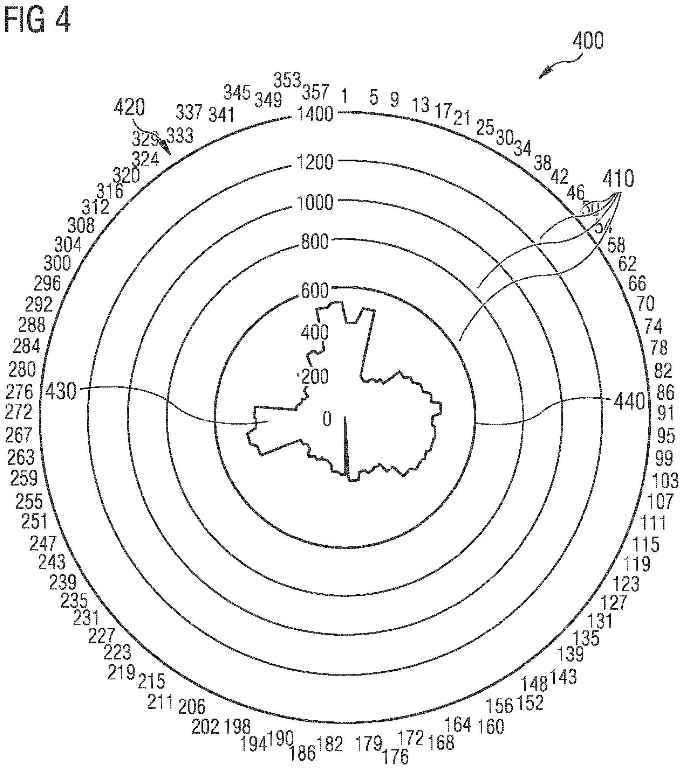

Fig. 4 depicts an exemplary dose distribution diagram 400 indicating a radiation dose rate arriving at a patient for different gantry angles of the gantry of a radiation apparatus and different dose rate settings of the radiation apparatus.

Fig. 4 depicts a dose distribution diagram 400 comprising a set of concentric circles 410, each circle 410 indicating a certain radiation dose (rate) arriving at a patient under various angle settings of the gantry. Points on a same circle may thus indicate that at each of these points, the same radiation dose arrives at a patient irrespective from the gantry angle similar to the concept of equipotential lines known from electrodynamics.

In the depicted example, the radiation apparatus (e.g., the radiation apparatus 140, 200 as described above) is operated at an exemplary maximum radiation dose rate of 1400 MUs for each of the gantry angles spanning from 0 to 357° indicated by the available maximum radiation dose rate circle 420.

However, to the aspects described herein, the available maximum radiation dose rate 420 seldomly arrives at the patient. Instead, only a portion of the available maximum radiation dose rate arrives at the patient, e.g., due to the limited rotation speed of the gantry.

The radiation dose which arrives at the patient is indicated by radiation dose line 430. In the depicted example, even though the dose delivered by the radiation apparatus is 1400 MUs, the dose arriving at the patient merely lies in between 0 MUs and 600 MUs and shows a significant dependence on the gantry angle.

It is therefore worthwhile, and in accordance with aspects of the present invention to decrease the maximum radiation dose rate to be transmitted by the radiation apparatus from the available maximum radiation dose rate (indicated by available maximum radiation dose rate circle 420) to an adapted maximum radiation dose rate (indicated by adapted maximum radiation dose rate circle 440). The adapted maximum radiation dose rate may be lower than the available maximum radiation dose rate (in the depicted example 600 MU) but still larger than the maximum required radiation dose according to the radiation treatment plan. Thus, even though the radiation apparatus may be operated at the decreased adapted maximum radiation dose rate, it may still be able to generate a radiation dose rate that allows providing the maximum radiation dose rate set out in the radiation treatment plan while facilitating the advantageous technical effects of the present invention as outlined elsewhere herein.

As another generic example, the modulator pulses may be provided at a rate of 360 Hz (thyratron may fire at 360 Hz) and an available maximum radiation dose rate may be 2400 MU/min. However, if it is determined that the adapted maximum radiation dose rate shall be 1200 MU/min, the rate at which the modulator pulses may be provided may be altered and decreased to 180 Hz. In such a case, the actual radiation therapy treatment for the patient may still remain unaltered as the maximum required dose rate for the actual radiation therapy (as per the determined radiation treatment plan) may still be below said reduced adapted maximum radiation dose rate of 1200 MU/min such as, e.g., 816 MU/min.

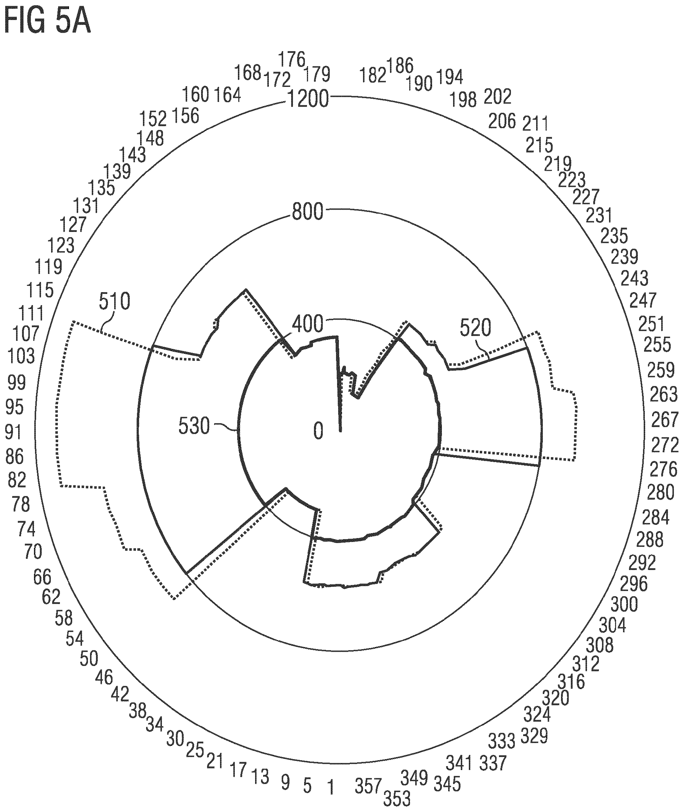

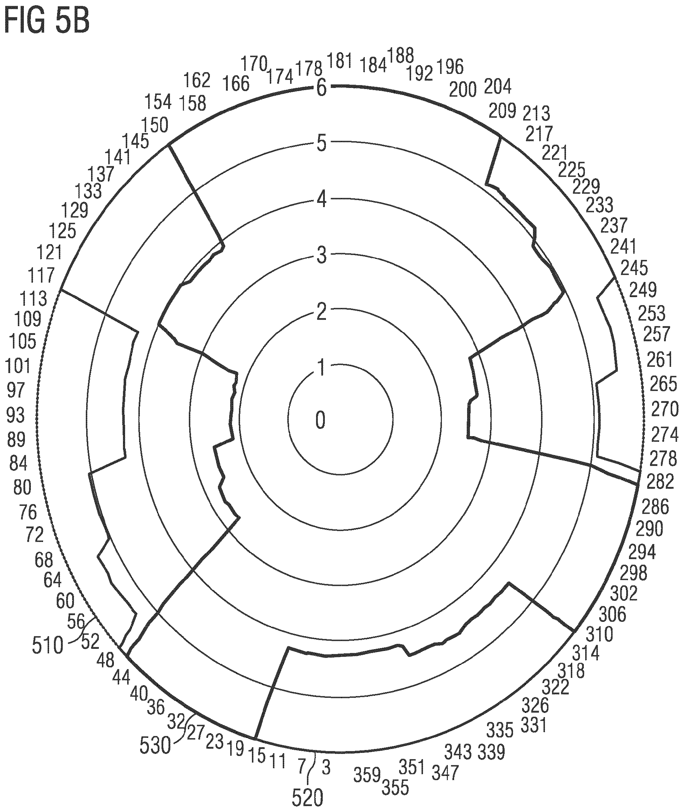

Figs. 5A and 5B depict the exemplary relationship between a dose rate arriving at a patient and a rotation speed of a gantry as it may be used for planning a radiation therapy.

Fig. 5A depicts exemplary maximum dose rates available for radiation therapy at a radiation apparatus for different rotation angles of the gantry. In the depicted example, dose rates of 1200 MU/min (510), 800 MU/min (520) and 400 MU/min (530) have been adjusted at the radiation apparatus (e.g., radiation apparatus 140, 200). Each of these dose rates may, e.g., represent an adapted maximum radiation dose rate.

Based thereon, Fig. 5A depicts how each of these dose rates at the radiation apparatus is translated to a dose rate arriving at a patient for various gantry angles. As can be derived from Fig. 5A, as the maximum dose at the radiation apparatus is decreased the radiation dose rate arriving at, e.g., a patient for different gantry angles decreases. As can be derived from Fig. 5A, as the maximum dose rate at the radiation apparatus is decreased from 1200 MU/min to 800 MU/min, the effective radiation dose rate difference arriving at the patient is smaller as compared to the exemplary case in which the maximum radiation dose rate at the radiation apparatus is decreased from 800 MU/min to 400 MU/min.

Fig. 5B shows the achievable maximum radiation dose rate at a patient for different rotation speeds of the gantry ranging from 1°/second to 6°/second.

Similar to Figs. 4 and 5A, the concentric circles indicate a region at which the same radiation dose rate may be achieved for certain gantry angles. That is, the innermost circle indicated by the numeral "1" indicates at rotation speed of the gantry of 1°/second. Any data points lying on said circle are associated with a rotation speed of the gantry of 1°/second.

Based thereon, it can be derived that for a rotation speed of, e.g., 2°/second a maximum dose rate of 400 MU/min can be obtained in a rotation angle range of 72° to 105°. However, if the rotation speed is increased to, e.g., 4.5°/second a maximum radiation dose rate of 800 MU/min can be obtained for approximately the same angular range of rotation angles of the gantry. If the rotation speed of the gantry is further increased to 6°/second, a maximum radiation dose rate of 1200 MU/min may be obtained.

To conclude, a higher rotation speed of the gantry may lead to higher radiation dose rates that may arrive at a patient. It is emphasized that generically there may not be a relationship between the radiation dose rate and the rotation speed of the gantry. However, during a radiation treatment planning procedure, a relationship may exist between a radiation dose rate and, e.g., different gantry angles due to mutual technical limitations (that is, if a certain rotation speed is selected, limitations may arise for the maximum radiation dose rate that may be transferred in a certain time interval).

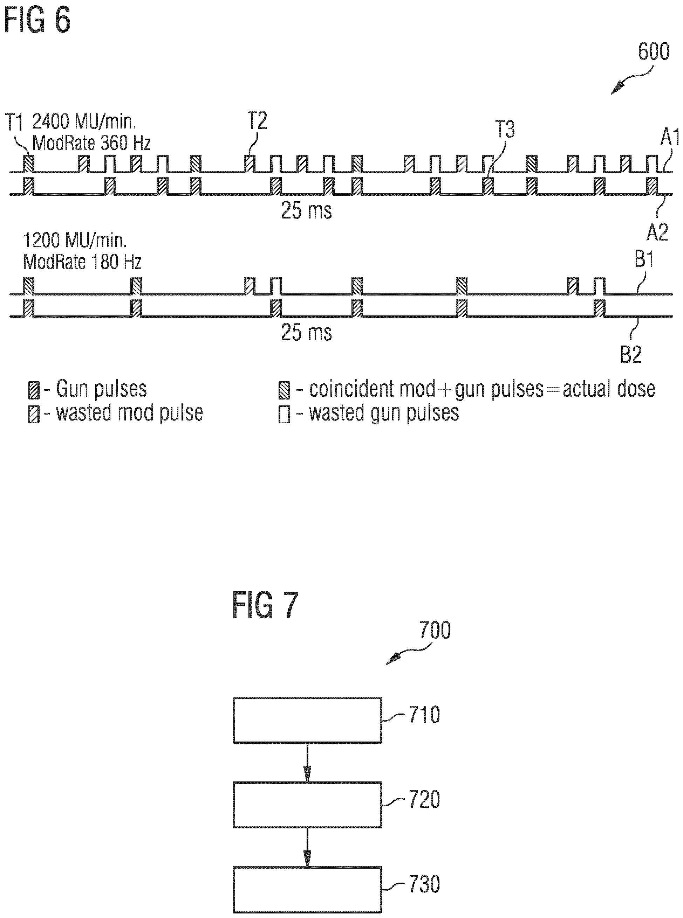

Fig. 6 depicts an exemplary comparison 600 between the temporal interplay of modulator pulses and gun pulses if the radiation apparatus is operated at its available maximum radiation dose rate (two top sequences A1, A2) and a respective interplay between modulator pulses and gun pulses if the radiation apparatus is configured to apply the adapted maximum radiation dose rate according to aspects of the present invention (two bottom sequences B1, B2). In particular, Fig. 6 intends to depict two exemplary scenarios how to supply a radiation dose rate of 800 MU/min at a radiation apparatus based on different combinations of modulator pulses and gun pulses.

In the depicted example (two top sequences A1, A2), the radiation apparatus is operated at an (exemplarily) available maximum radiation dose rate of 2400 MU/min and a modulator pulse rate of 360 Hz (360 modulator pulses/s). That is, in the depicted example, in the depicted time interval of 25 ms, 12 gun pulses are executed.

The first sequence A1 depicts an exemplary sequence of a number K of modulator pulses of a first temporal sequence at which charged particles (e.g., electrons) may be accelerated in the acceleration stage of the radiation apparatus. The second first sequence A2 of the two first temporal sequences depicts an exemplary sequence of a number M of gun pulses of a first temporal sequence at which charged particles (e.g., electrons) may enter the acceleration stage.

If a gun pulse of the first temporal sequence coincides with a modulator pulse of the first temporal sequence, a respective charged particle which has entered the acceleration stage may successfully be accelerated. This scenario is exemplarily depicted as time stamp T1.

However, if a modulator pulse occurs but is not at least partially overlapped by a gun pulse in the time domain, the respective modulator pulse may be considered as a wasted modulator pulse. This scenario is exemplarily depicted as time stamp T2.

In some examples, a gun pulse may at least partially coincide with a modulator pulse but no acceleration of the respective charged particle occurs. In such an example, the situation may be considered as a wasted gun pulse as charged particles may enter the acceleration stage but are not accelerated any further. This scenario is exemplarily depicted as time stamp T3.

With a view to sequences A1 and A2, it can be derived that every third gun pulse (sequence A2) coincides with a modulator pulse (sequence A1). That is, effectively a third of the gun pulses is used for providing a certain dose rate. In other words, the effective radiation dose rate leaving the radiation apparatus is only a third of 2400 MU/min, namely 800 MU/min.

The two bottom sequences B1 and B2 depict the exemplary example in which the radiation apparatus is operated at an adapted maximum radiation dose rate of 1200 MU/min and a modulation rate of 180 Hz (180 modulator pulses/s), i.e., at a dose rate that is lower than the available maximum dose rate expressed as a first second sequence B1 and a second sequence B2. Sequence B2 thus only comprises 6 gun pulses within the depicted interval of 25 ms.

As a result, less charged particles are injected into the acceleration stage (as compared to the scenario depicted in sequences A1 and A2) and less potential acceleration opportunities for charged particles are provided due to the reduced frequency of the gun pulses as compared to the two top sequences A1 and A2.

It is further derivable from sequences B1 and B2 that only two out of three gun pulses coincide with a respective modulator pulse. As a consequence, the effectively transmitted radiation dose rate is 2/3 of 1200 MU/min, namely 800 MU/min.

As depicted in sequences A1 and A2 as compared to sequences B1 and B2, the radiation dose rate of 800 MU/min may be obtained from a combination of different parameters of a radiation apparatus.

Fig. 7 shows an exemplary computer-implemented method 700 for radiation treatment planning according to an aspect of the present invention.

In step 710, a radiation treatment plan comprising a number N, with N ≥ 1, of radiation sequences to be applied to a patient is determined, each radiation sequence being associated with a radiation dose.

In step 720, a maximum radiation dose rate of the radiation treatment plan is determined, the maximum radiation dose rate being associated with one of the N radiation sequences.

In step 730, a radiation apparatus to apply an adapted maximum radiation dose rate in accordance with the determined maximum radiation dose rate is configured, wherein the adapted maximum radiation dose rate is less than an available maximum radiation dose rate at the radiation apparatus.

The energy-saving effects may depend on the actual type of treatment. That is, the biggest impact is expected to be exhibited on conventionally fractionated flattening filter free (FFF) RapidArc (volumentric intensity arc therapy, VMAT) treatments.

Other radiation therapies are either made at full dose rate or close to that such as, e.g., intensity modulated radiation therapy (IMRT), standard photons, hypo-fractioned, stereotactic body radiation therapy (SBRT), etc.

For typical radiation therapy sessions (typically 20-30 sessions per patient), fractionated FFF RapidArc (VMAT) treatments may be performed that may lead to an on average reduction of 50% of the pulses (modulation and/or gun pulses). Standard photons VMAT treatments may be up to 45% of a total treatment time which may progressively be changed to FFF.

In a typical radiation apparatus, a difference in power consumption when cutting an available maximum dose rate to half of it may, e.g., be 8.8 kW. Annually, 7 million US-dollars may be spent on Thyratrons.

Based on the method described herein, in a worst-case scenario, energy savings on the order of 8.9 million US-dollars, 14.2 ktons of CO2 may be achieved while 120k trees may be saved whereas thyratron-related savings of 9.3 MM$ are expected. However, in a best-case scenario, energy savings of 23 million US-dollars, 37.6 ktons of CO2, 212k trees or the equivalent of 84.7 billion smartphone charging cycles may be saved. A thyratron-related saving of 22.5 million US-dollars may be expected.

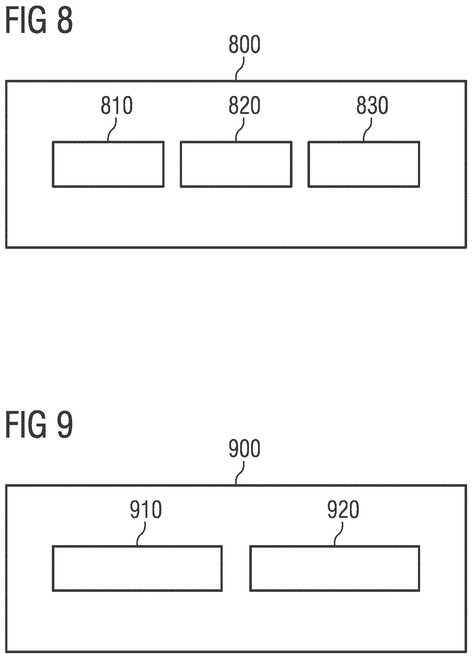

Fig. 8 shows an exemplary computer-implemented apparatus 800 for radiation treatment planning according to an aspect of the present invention. The apparatus 800 includes a determining unit 810, a further determining unit 820 and a configuring unit 830.

Determining unit 810 may be configured to determine a radiation treatment plan comprising a number N, with N ≥ 1, of radiation sequences to be applied to a patient, each radiation sequence being associated with a radiation dose

Further determining unit 820 may be configured to determine a maximum radiation dose rate of the radiation treatment plan, the maximum radiation dose rate being associated with one of the N radiation sequences.

Configuring unit 830 may be configured to configure a radiation apparatus to apply an adapted maximum radiation dose rate in accordance with the determined maximum radiation dose rate, wherein the adapted maximum radiation dose rate is less than an available maximum radiation dose rate at the radiation apparatus.

Fig. 9 shows an exemplary system 900 for radiation treatment planning according to an aspect of the present invention. The system 900 includes a computer-implemented apparatus 910 and a computer program product 920.

Computer-implemented apparatus 910 may be configured as described elsewhere herein.

Computer program product 920 may be configured as described elsewhere herein.

Although the present invention has been described in accordance with preferred embodiments, it is obvious for the person skilled in the art that modifications are possible in all embodiments.

Independent of the grammatical term usage, individuals with male, female or other gender identifies are included with the term.

### List of Reference

- 100: system
- 110: computer-implemented apparatus
- 111: determining unit
- 112: further determining unit
- 113: configuring unit
- 120: user interface
- 130: remote unit
- 140: radiation apparatus
- 150: cloud service
- 160: patient
- 200: radiation apparatus
- 210: gun
- 220: acceleration stage
- 221: RF input unit
- 222: acceleration electrode
- 223: solenoid
- 230: beam bending unit
- 240: beam shaping unit
- 300: exemplary pulses
- 400: dose distribution diagram
- 410: circle of constant radiation dose (rate)
- 420: available maximum radiation dose rate circle
- 430: radiation dose line
- 440: adapted maximum radiation dose rate circle
- 510: first dose rate
- 520: second dose rate
- 530: third dose rate
- 600: exemplary pulse comparison
- 700: computer-implemented method
- 710: step
- 720: step
- 730: step
- 800: computer-implemented apparatus
- 810: determining unit
- 820: further determining unit
- 830: configuring unit
- 900: system
- 910: computer-implemented apparatus
- 920: computer program product
- T1: time stamp
- T2: time stamp
- T3: time stamp
- A1: first temporal sequence
- A2: second first temporal sequence
- B1: first second temporal sequence
- B2: second temporal sequence

## Claims

1. A computer-implemented method (700) for radiation treatment planning, comprising:
determining (710) a radiation treatment plan comprising a number N, with N ≥ 1, of radiation sequences to be applied to a patient (160), each radiation sequence being associated with a radiation dose rate;
determining (720) a maximum radiation dose rate of the radiation treatment plan, the maximum radiation dose rate being associated with one of the N radiation sequences; and
configuring (730) a radiation apparatus (140; 200) to apply an adapted maximum radiation dose rate in accordance with the determined maximum radiation dose rate, wherein the adapted maximum radiation dose rate is less than an available maximum radiation dose rate at the radiation apparatus (140; 200).

2. The computer-implemented method according to claim 1,
wherein each of the N radiation sequences is associated with a gantry angle of a set of gantry angles of the radiation apparatus (140; 200).

3. The computer-implemented method according to claim 1 or 2,
wherein the determining (710) of the N radiation sequences comprises:
determining a first number M, with M ≥ 1, of gun pulses defining a first temporal sequence (A2) at which charged particles enter an acceleration stage (220) of the radiation apparatus (140; 200); and
determining a first number K, with K ≥ 1, of modulator pulses defining a first temporal sequence (A1) at which the charged particles are accelerated in the acceleration stage of the radiation apparatus (140; 200).

4. The computer-implemented method according to claim 3,
wherein the configuring (730) comprises:
determining a second number M of gun pulses defining a second temporal sequence (B2) at which charged particles enter the acceleration stage and/or a second number N of modulator pulses defining a second temporal sequence (B1) at which the charged particles are accelerated, wherein the second temporal sequence (B2) of gun pulses and/or the second temporal sequence (B1) of modulator pulses comprises less pulses in a predefined time interval than the respective first temporal sequence (A2) of gun pulses and/or the first temporal sequence (A1) of modulator pulses.

5. The computer-implemented method according to claim 4,
wherein the second number M of gun pulses defining a second temporal sequence (B2) at which charged particles enter the acceleration stage (220) is lower as compared to the first number M of gun pulses defining a first temporal sequence (A2) at which charged particles enter the acceleration stage (220) and/or wherein the second number K of modulator pulses defining a second temporal sequence (B1) at which charged particles are accelerated is lower as compared to the first number K of modulator pulses defining a first temporal sequence (A1) at which charged particles are accelerated.

6. The computer-implemented method according to one of claims 3-5,
wherein an acceleration of charged particles that enter the acceleration stage (220) occurs when a gun pulse of the number M of gun pulses at least partially overlaps in the time domain with a modulator pulse of the number K of modulator pulses.

7. The computer-implemented method according to one of claims 1-6,
wherein determining (720) the maximum radiation dose rate of the radiation treatment plan comprises determining a particular temporal sequence of gun pulses and/or a temporal sequence of modulator pulses that causes the determined maximum radiation dose rate.

8. The computer-implemented method according to one of claims 1-7,
wherein each radiation dose rate to be applied to a patient (160) according to the radiation treatment plan and as derived from each of the N radiation sequences is equal to a radiation dose rate associated with the adapted maximum dose rate the radiation apparatus (140; 200) is configured with.

9. The computer-implemented method according to one of claims 1-8, further comprising:
configuring the radiation apparatus (140; 200) to apply the radiation sequence in accordance with the configured adapted maximum radiation dose rate and the determined radiation treatment plan.

10. The computer-implemented method according to one of claims 1-9,
wherein the configured adapted maximum radiation dose rate of the radiation apparatus (140; 200) is at least lower by 30%, preferably by at least 40%, more preferably by at least 60% and most preferably by at least 70% than the available maximum radiation dose rate of the radiation apparatus (140; 200).

11. The computer-implemented method according to one of claims 1-10,
wherein the radiation treatment planning is performed for a radiation therapy of a breast, a prostate, a head and/or a neck of the patient.

12. A computer program product (920) comprising instructions which, when the program is executed by a computer, cause the computer to carry out the method according to any of the claims 1-11.

13. A computer-implemented apparatus (800) for radiation treatment planning, comprising:
a determining unit (810) for determining a radiation treatment plan comprising a number N, with N ≥ 1, of radiation sequences to be applied to a patient (160), each radiation sequence being associated with a radiation dose rate;
a further determining unit (820) for determining a maximum radiation dose rate of the radiation treatment plan, the maximum radiation dose rate being associated with one of the N radiation sequences; and
a configuring unit (830) for configuring a radiation apparatus (140; 200) to apply an adapted maximum radiation dose rate in accordance with the determined maximum radiation dose rate, wherein the adapted maximum radiation dose rate is less than an available maximum radiation dose rate at the radiation apparatus (140; 200).

14. The computer-implemented apparatus of claim 13, further comprising:
an execution unit for executing the computer-implemented method according to one of the claims 1-11 and/or
a further execution unit for executing the computer program product according to claim 12.

15. A system (900) for radiation treatment planning, comprising:
the computer-implemented apparatus (910) according to one of the claims 13 or 14; and
the computer program product (920) according to claim 12.
